# EUROPEAN PATENT APPLICATION

(11) **EP 4 715 089 A1**
(43) Date of publication of application: **25.03.2026**
(21) Application number: 24806250.7
(22) Date of filing: 15.04.2024
(51) Int. Cl.: C25B 1/04, C25B 9/00, C25B 15/00, A61M 16/10, A61M 16/16

(54) **HYDROGEN GENERATING DEVICE PROVIDED WITH SOUND INSULATION COVER AND HYDROGEN GENERATING DEVICE PROVIDED WITH NOVEL POWER MODULE**

(30) Priority: 18.05.2023 CN 202310560363
(71) Applicant: Lin, Hsin-Yung, Taoyuan, Taiwan 33341 (TW)
(72) Inventor: Lin, Hsin-Yung, Taoyuan, Taiwan 33341 (TW)
(74) Representative: Wang, Bo
(86) International application number: PCT/CN2024/087648
(87) International publication number: WO 2024/234893

(57) **Abstract**

Ahydrogen generator includes a water tank configured to contain electrolysis water, an electrolysis module disposed in the water tank and configured to electrolyze the electrolysis water to generate a gas comprising hydrogen, a humidifying module having a humidifying chamber configured to contain supplement water, a diffusing device disposed in the humidifying module and configured to diffuse the gas comprising hydrogen, and a sound-proof shield disposed in the humidifying module and including a sound-proof cavity, a connecting tube communicating the water tank and the diffusing device, and a gas outlet. The gas comprising hydrogen flows through the connecting tube and the diffusing device to the supplement water in the sound-proof cavity, and then passes through the gas outlet to the humidifying chamber. The sound-proof shield blocks sound generated by the gas comprising hydrogen flowing in the device, thereby improving user experience.

## Description

### BACKGROUND OF THE INVENTION

### 1. Field of the invention

The present invention relates to a hydrogen generator, and more particularly to a hydrogen generator provided with a sound-proof shield and a new power module.

### 2. Description of the prior art

People always pay much attention to human life, so many medical technologies are developed to fight disease and keep people alive. Most of the past medical treatment methods are passive; namely, when a disease occurs, the medical treatment, such as surgery, medication and chemotherapy, radiation or recovery from chronic illness, rehabilitation and correction are given. However, in recent years, many medical experts have increasingly concentrated on preventive medical methods, such as healthy food research, genetic disease screening and disease prevention, and even have actively taken initiatives to prevent possible future diseases. In addition, in order to extend life expectancy of human beings, numerous anti-aging and anti-oxidant technologies, including skin care products and foods/anti-oxidant drugs, have been gradually developed and widely adopted by the public.

Researches show that the body's unstable oxygen gas (O+), also known as free radicals (harmful free radicals), caused by various reasons (such as disease, diet, environment or living habits), can mix with inhaled hydrogen gas to form water and be excreted by the body. Namely, by indirectly reducing the number of free radicals in the human body, the reduction of acidic physique is achieved to become healthy alkaline physique, which can resist oxidation and anti-aging, as well as achieve the effects of eliminating chronic diseases and beauty care. Moreover, by increasing the amount of hydrogen gas inhaled, the increase of the time spent on inhaling hydrogen gas (for example, inhaling hydrogen gas during sleeping time) may also efficiently improve the effect of hydrogen gas inhalation.

The hydrogen generators on the market typically generate hydrogen by electrolyzing electrolyte-containing water, and the gas comprising hydrogen is filtered and humidified by a humidifying module so that it can be more easily inhaled by a user. However, when the gas comprising hydrogen flows in the humidifying module and reaches the interface between water and air in the humidifying module, ejection of the gas comprising hydrogen disturbs and vibrates the water surface, thereby generating sound. Furthermore, disturbance and vibration of the water surface are transmitted to an inner wall of the humidifying module, which may cause additional noise. When the user operates the hydrogen generator during sleep, sound generated by the flowing gas comprising hydrogen degrades user experience. In addition, during operation of the hydrogen generator, electronic components on a circuit board generate heat. When an overall operating temperature of the hydrogen generator becomes excessively high, an electrolysis module that electrolyzes water to generate the gas comprising hydrogen suffers reduced electrolysis efficiency due to the elevated temperature.

### SUMMARY OF THE INVENTION

Therefore, the present invention provides a new hydrogen-generating device to address problems of the prior art.

In one embodiment, a hydrogen generator with a sound-proof shield includes a water tank, an electrolysis module, a humidifying module, a diffusing device, and a sound-proof shield. The water tank has a space configured to contain electrolysis water. The electrolysis module is configured to receive the electrolysis water from the water tank and electrolyze the electrolysis water to generate and output a gas comprising hydrogen. The humidifying module is stacked above the water tank and configured to humidify the gas comprising hydrogen. The humidifying module has a humidifying chamber configured to contain supplement water. The diffusing device is disposed in the humidifying chamber and configured to diffuse the gas comprising hydrogen output by the electrolysis module. The sound-proof shield is disposed in the humidifying chamber and has a sound-proof cavity configured to accommodate the diffusing device. The sound-proof shield comprises a connecting tube isolated from the sound-proof cavity, and a top portion of the sound-proof shield comprises a gas outlet communicating the sound-proof cavity with the humidifying chamber. The connecting tube is in fluid communication with the diffusing device and is configured to guide the gas comprising hydrogen generated by the electrolysis module into the diffusing device. The gas comprising hydrogen flows through the diffusing device to the sound-proof cavity, and the gas comprising hydrogen in the sound-proof cavity then flows through the gas outlet to the humidifying chamber.

The hydrogen generator with the sound-proof shield further includes a filter sheet disposed outside the sound-proof shield and configured to cover the gas outlet.

The hydrogen generator with the sound-proof shield further includes a securing structure configured to contact and abut against the filter sheet, thereby attaching the filter sheet to the sound-proof shield and covering the gas outlet.

A water level of the supplement water in the humidifying chamber is lower than a mounting position of the filter sheet.

An outer wall of the sound-proof shield and an inner wall of the humidifying module define a gap.

The diffusing device further includes a plurality of micro-holes, which allow the gas comprising hydrogen to pass through the micro-holes into the sound-proof cavity and form a plurality of micro bubbles in the supplement water.

The hydrogen generator with the sound-proof shield further includes a filter channel device, a condenser, and an integrated flow channel device. The filter channel device is configured to receive and filter the gas comprising hydrogen. The condenser is stacked above the water tank and connected to the filter channel device, and is configured to receive and condense the gas comprising hydrogen output by the filter channel device. The integrated flow channel device is stacked above the water tank and communicates the condenser and the humidifying module. The integrated flow channel device is configured to receive the condensed gas comprising hydrogen and output the gas comprising hydrogen to the humidifying module.

The integrated flow channel device includes a supplement water channel, and the sound-proof shield includes a supplement water passage communicating the supplement water channel and the sound-proof cavity. The supplement water is replenished into the sound-proof cavity through the supplement water channel and the supplement water passage.

The hydrogen generator with the sound-proof shield further includes a power module disposed above the water tank. The power module is electrically connected to the electrolysis module and is configured to supply electric energy to the electrolysis module so that the electrolysis module electrolyzes the electrolysis water.

The power module includes a base in a cuboid shape and a plurality of heat-dissipation fins. The base has a heat-dissipation channel and an inner surface, and the heat-dissipation fins extend inward from the inner surface into the heat-dissipation channel.

The hydrogen generator with the sound-proof shield further includes an atomizer in gas communication with the humidifying module. The atomizer receives the gas comprising hydrogen from the humidifying module and selectively generates an atomized gas to be mixed with the gas comprising hydrogen to form a health-care gas.

In one embodiment, a hydrogen generator with a new power module includes a water tank, an electrolysis module, a humidifying module, a sound-proof shield, and a power module. The water tank has a space configured to contain electrolysis water. The electrolysis module is configured to receive the electrolysis water from the water tank and electrolyze the electrolysis water to generate and output a gas comprising hydrogen. The humidifying module is stacked above the water tank and configured to humidify the gas comprising hydrogen. The humidifying module has a humidifying chamber configured to contain supplement water. The sound-proof shield is disposed in the humidifying chamber and has a sound-proof cavity. The gas comprising hydrogen output by the electrolysis module is guided into the sound-proof cavity. The power module is disposed above the water tank and electrically connected to the electrolysis module. The power module is configured to supply electric energy to the electrolysis module and includes a base, a first circuit board, a second circuit board, and a fan. The base has a first outer surface and a second outer surface opposite to each other and includes a heat-dissipation channel located between the first outer surface and the second outer surface. The first circuit board and the second circuit board are respectively disposed on the first outer surface and the second outer surface. The fan is disposed at one end of the base and is configured to introduce ambient air into the heat-dissipation channel.

The base has a first inner surface and a second inner surface opposite to each other and includes a plurality of heat-dissipation fins. The heat-dissipation fins respectively extend inward from the first inner surface and the second inner surface into the heat-dissipation channel.

The electrolysis module is disposed in the space of the water tank. The water tank includes a tank body and a cover, and the electrolysis module has an electrolysis module body. The cover includes a first fixing portion, and the electrolysis module body includes a second fixing portion toward the cover and connected to the first fixing portion, thereby suspending the electrolysis module from the cover.

The cover includes a first positioning structure, and the electrolysis module body includes a second positioning structure corresponding to the first positioning structure. When the electrolysis module and the cover are connected through the first fixing portion and the second fixing portion such that the electrolysis module is suspended from the cover, the first positioning structure is coupled to the second positioning structure.

The tank body forms a plurality of third positioning structures at a bottom of the space, and a bottom of the electrolysis module body includes a plurality of fourth positioning structures corresponding to the third positioning structures. When the electrolysis module is disposed in the space, the third positioning structures are movably coupled to the fourth positioning structures.

In summary, the hydrogen generator with the sound-proof shield can block sound generated by the gas comprising hydrogen flowing in the device through the sound-proof shield, thereby improving user experience. In addition, the hydrogen generator with the sound-proof shield includes a filter channel device and a condenser that are individually detachable, thereby improving convenience and installation efficiency. Moreover, the condenser of the hydrogen generator with the sound-proof shield can effectively increase a condensation path and heat-dissipation performance through a single extendable flow path and heat-dissipation elements, thereby enhancing condensation and filtering efficiency. Furthermore, the hydrogen generator with the sound-proof shield includes a new power module, which dissipates heat from the circuit boards effectively through the base having the heat-dissipation channel, thereby improving operating efficiency.

### BRIEF DESCRIPTION OF THE APPENDED DRAWINGS

Fig. 1 is a schematic structural diagram of a hydrogen generator with a sound-proof shield according to an exemplary embodiment of the present invention.
Fig. 2 is a functional block diagram of the hydrogen generator with the sound-proof shield of Fig. 1.
Fig. 3 is an exploded view of the hydrogen generator with the sound-proof shield of Fig. 1.
Fig. 4A is an exploded view of the water tank of Fig. 1.
Fig. 4B is a schematic structural diagram of the humidifying module of Fig. 1.

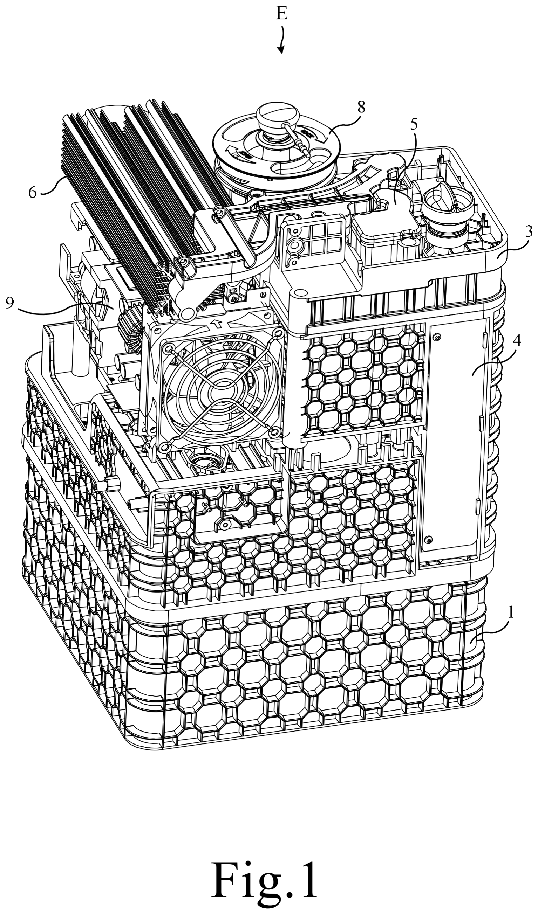

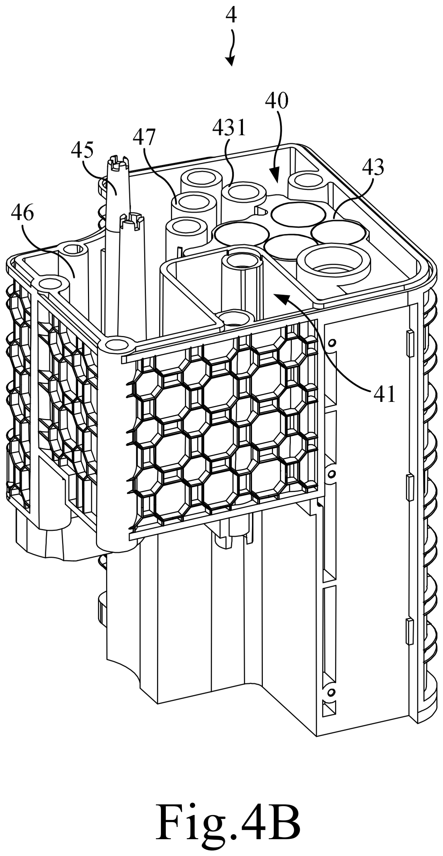

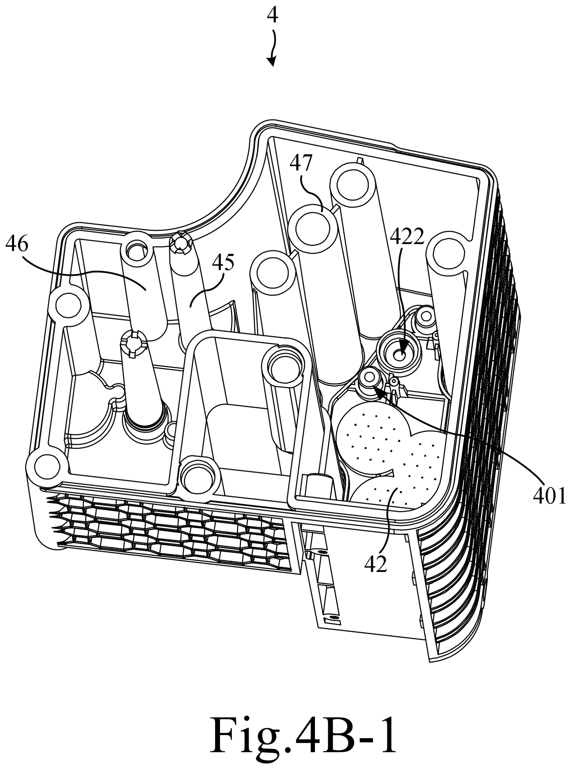

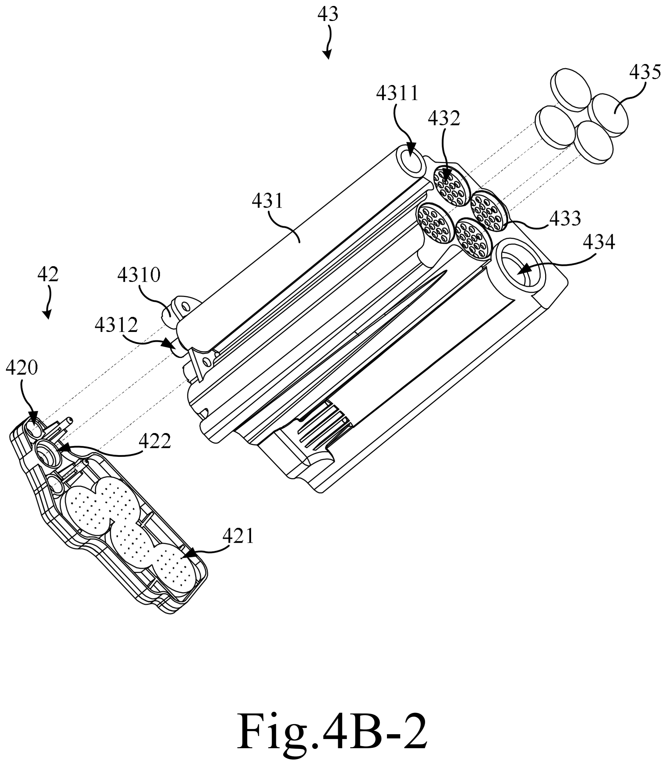

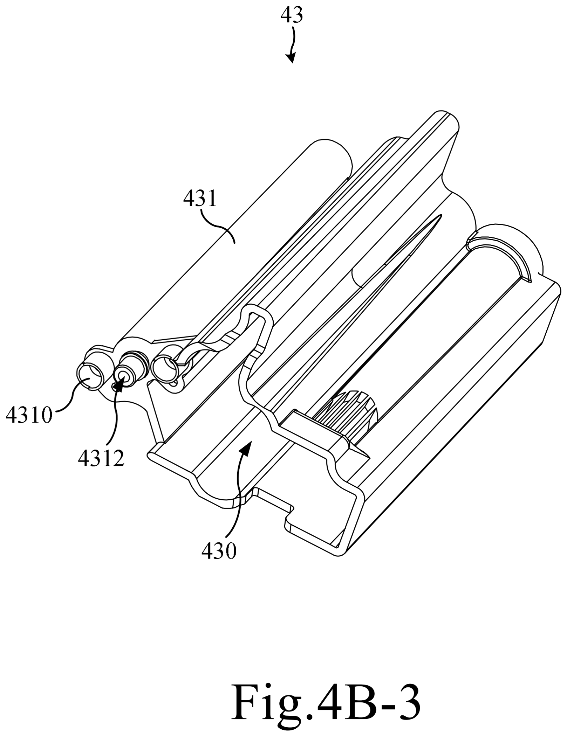

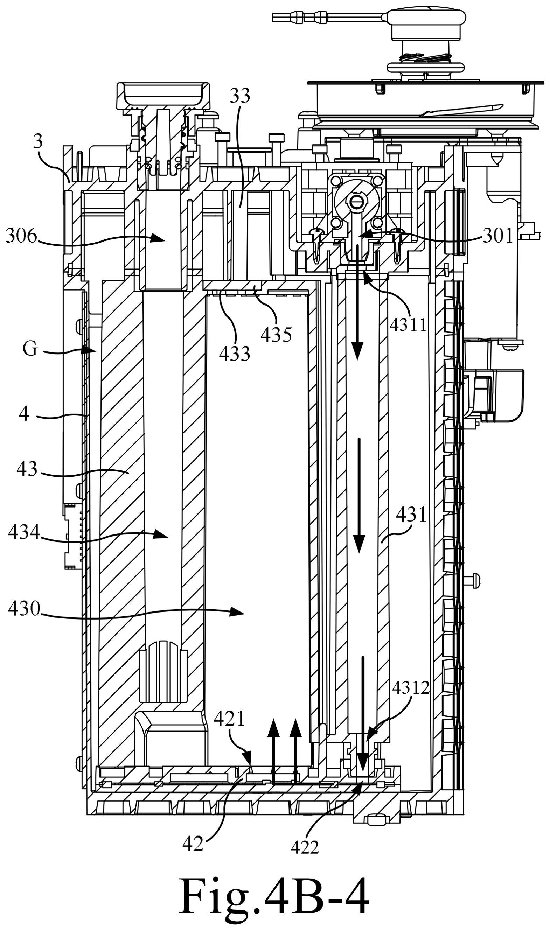

Fig. 4B-1 is a schematic structural diagram of the humidifying module of Fig. 1 from another perspective.
Fig. 4B-2 is an exploded view of the sound-proof shield and the diffusing device.
Fig. 4B-3 is a schematic structural diagram of the sound-proof shield from another perspective.
Fig. 4B-4 is a cross-sectional view of the integrated flow channel device, the humidifying module, the sound-proof shield, and the diffusing device.

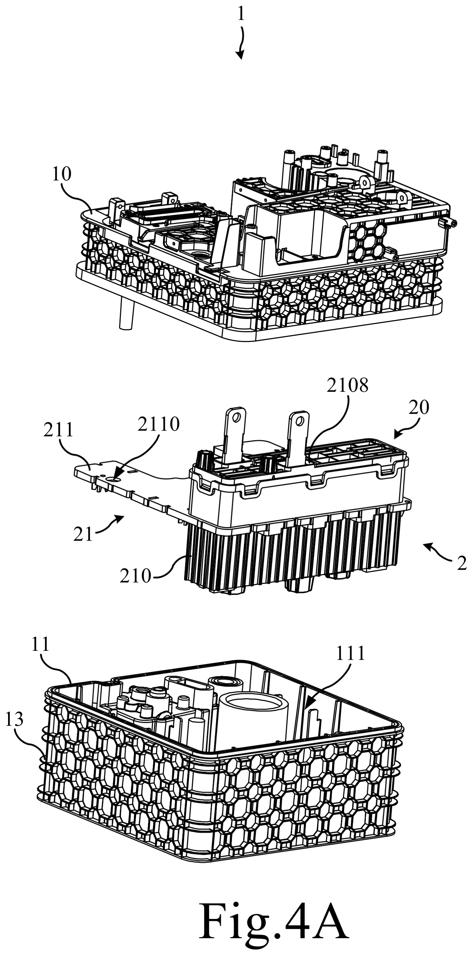

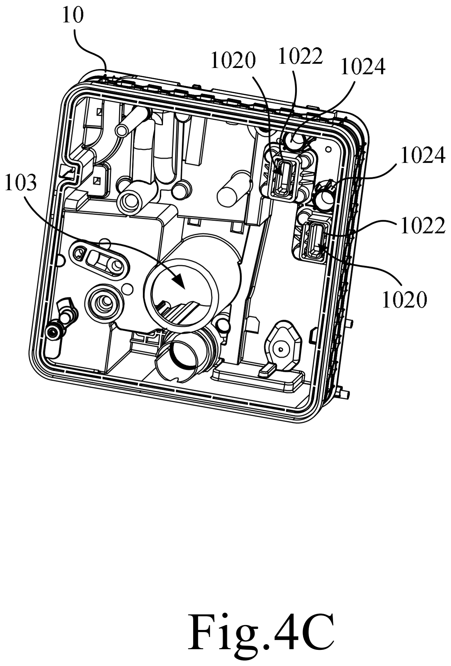

Fig. 4C is a schematic view of the cover of Fig. 4A from another perspective.

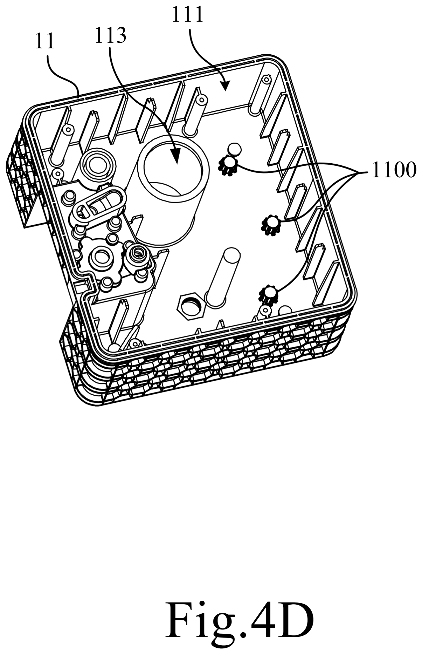

Fig. 4D is a schematic view of the tank body of Fig. 4A from another perspective.

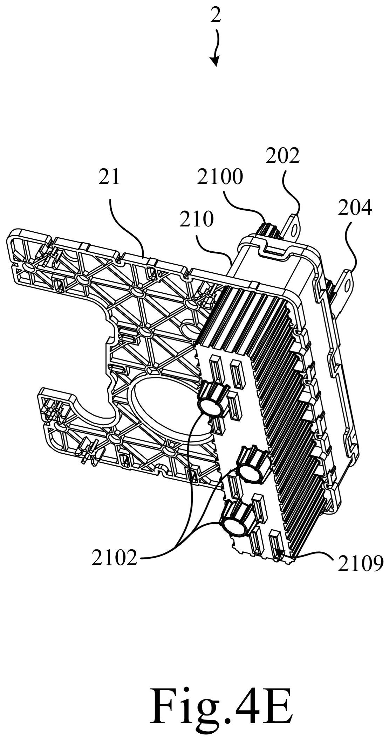

Fig. 4E is a schematic view of the electrolysis module of Fig. 4A from another perspective.

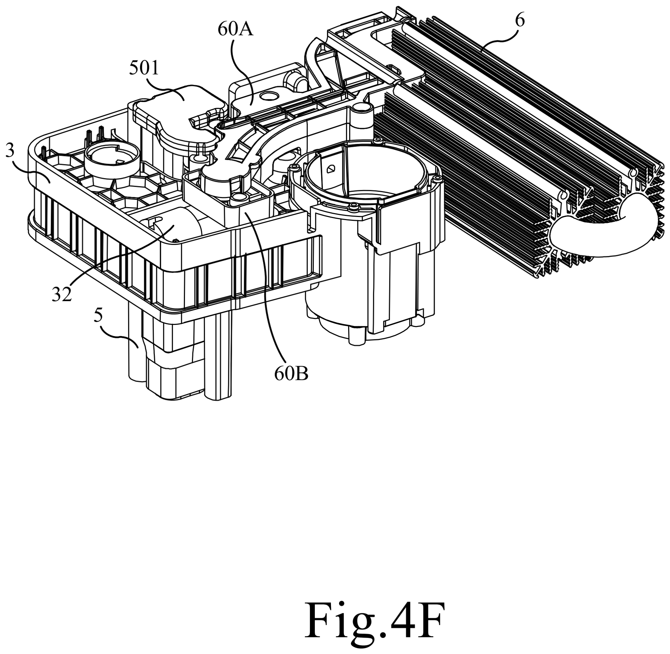

Fig. 4F is an assembled view of the integrated flow channel device, the filter channel device, and the condenser of Fig. 1.

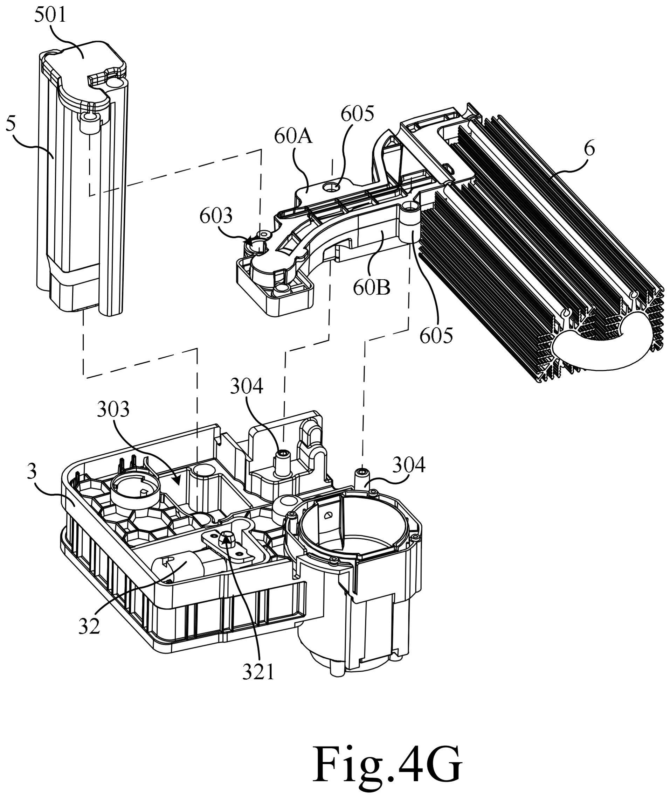

Fig. 4G is an exploded view of the integrated flow channel device, the filter channel device, and the condenser of Fig. 1.

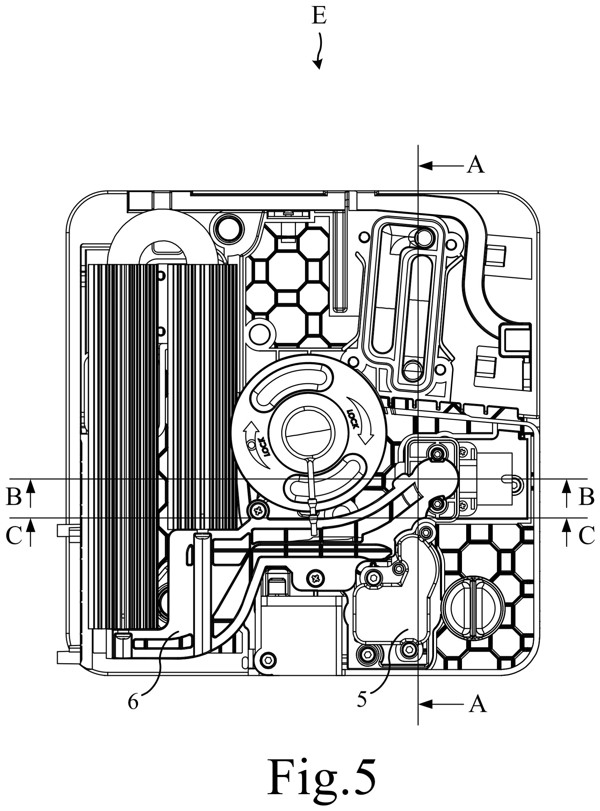

Fig. 5 is a schematic structural diagram of the hydrogen generator with the sound-proof shield of Fig. 1 from another perspective.

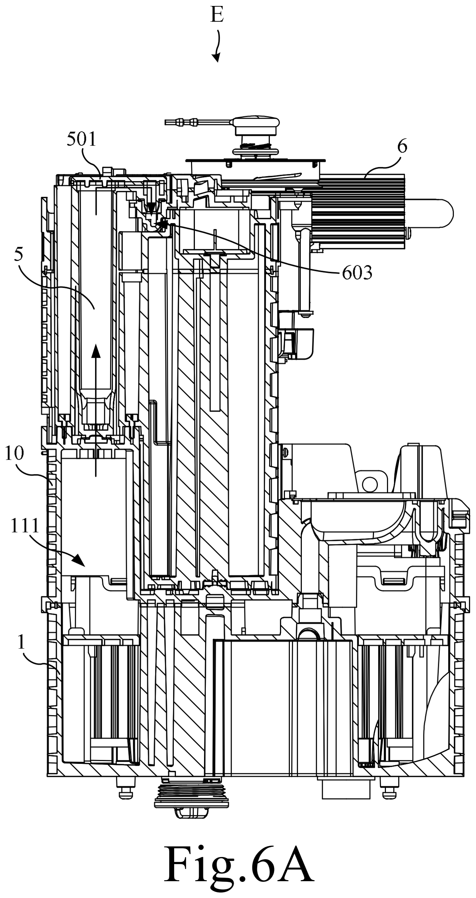

Fig. 6A is a cross-sectional view taken along line A-A of Fig. 5.

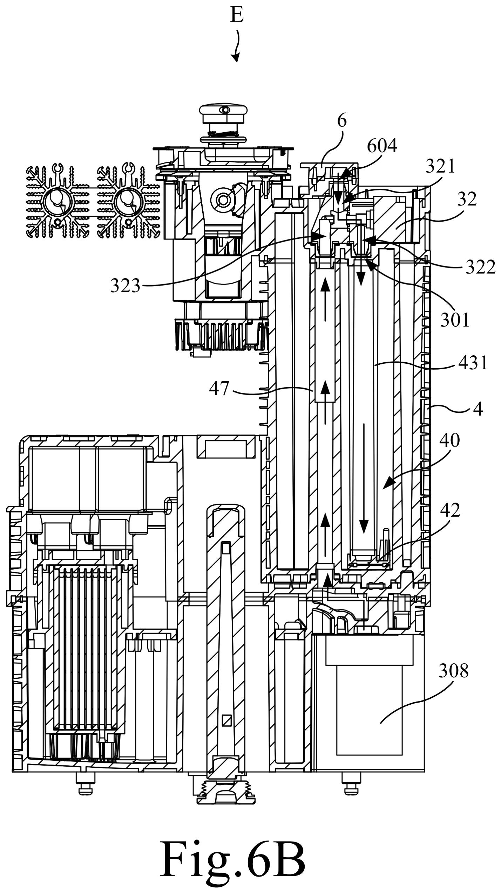

Fig. 6B is a cross-sectional view taken along line B-B of Fig. 5.

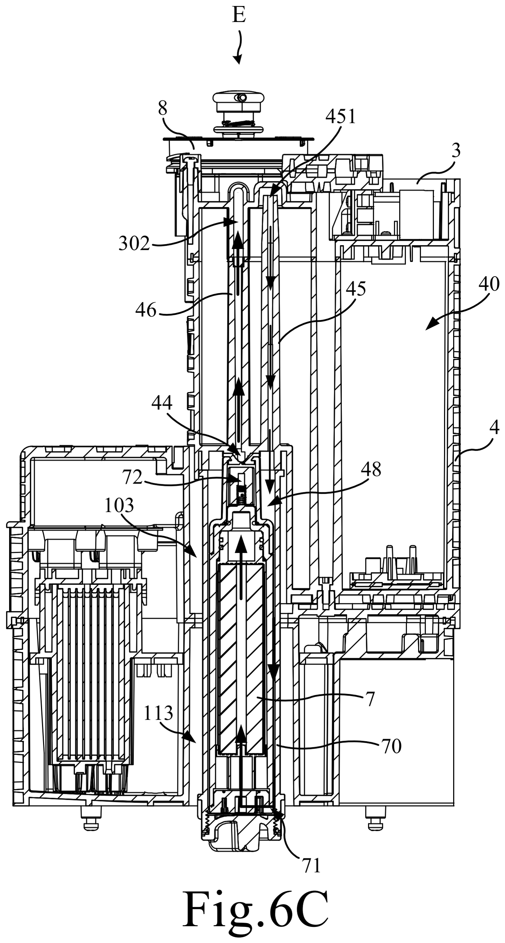

Fig. 6C is a cross-sectional view taken along line C-C of Fig. 5.

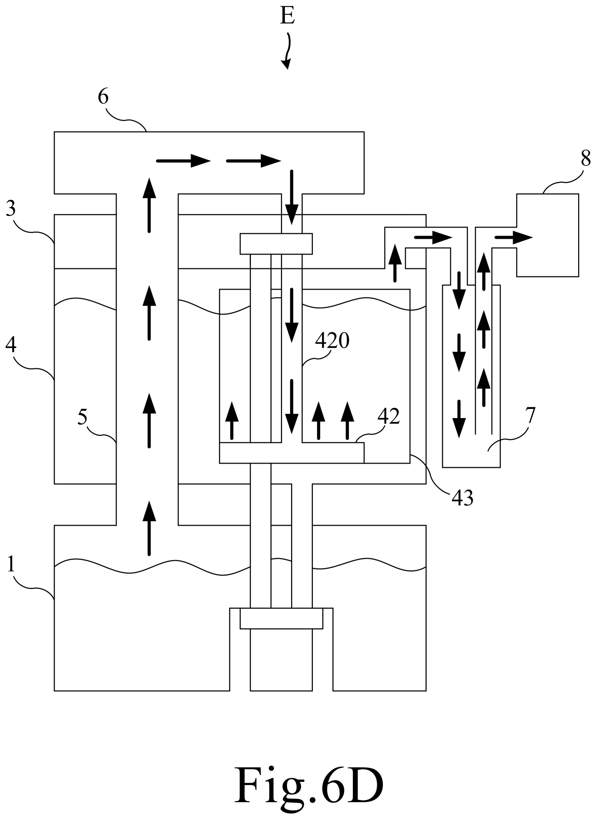

Fig. 6D is a simplified schematic diagram of a gas flow of the hydrogen generator with the sound-proof shield according to an exemplary embodiment of the present invention.

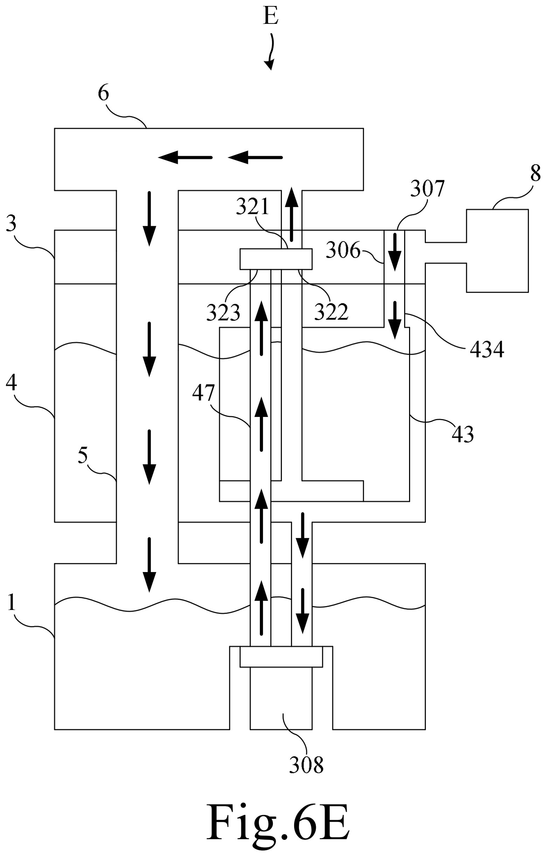

Fig. 6E is a simplified schematic diagram of a supplement water flow of the hydrogen generator with the sound-proof shield according to an exemplary embodiment of the present invention.

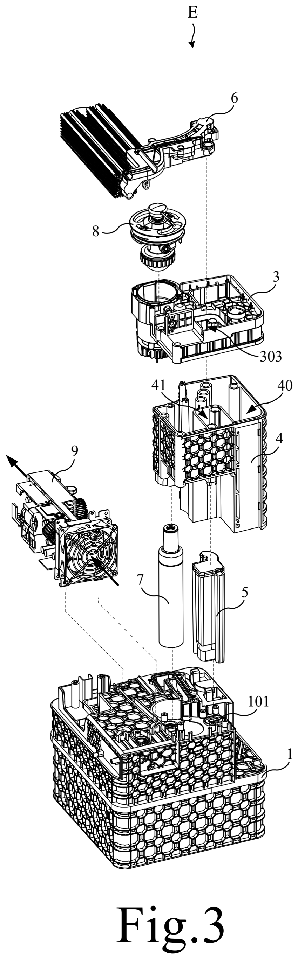

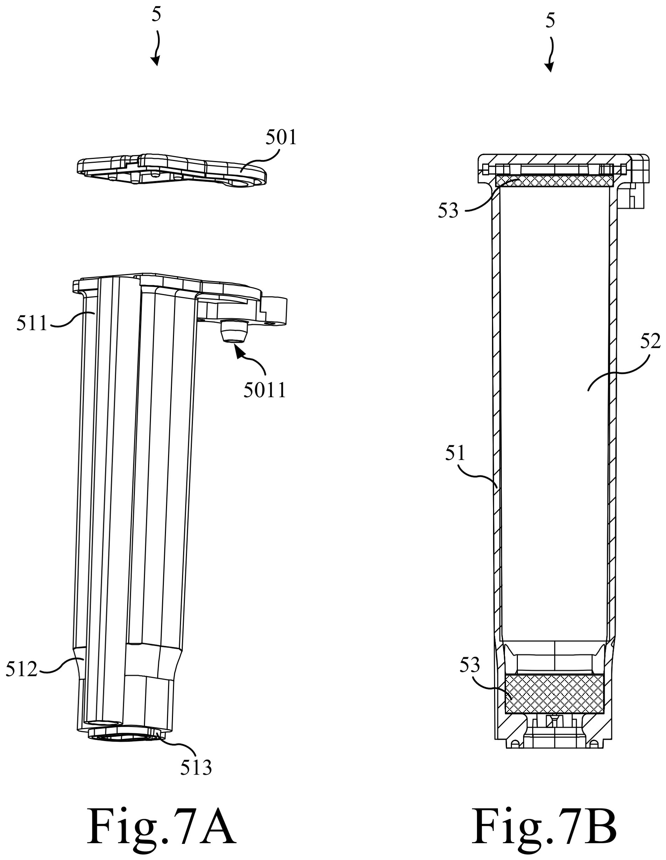

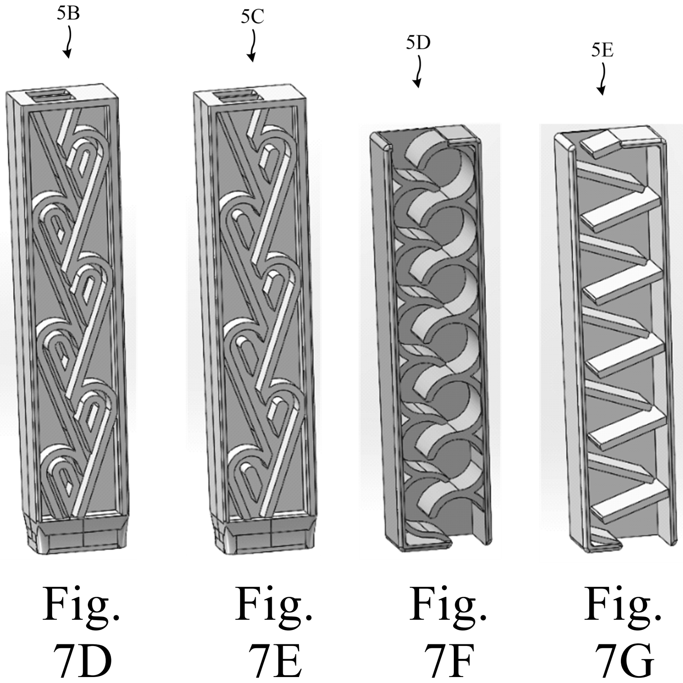

Fig. 7A is an exploded view of the filter channel device of Fig. 3.
Fig. 7B is a cross-sectional view of the filter channel device of Fig. 3.

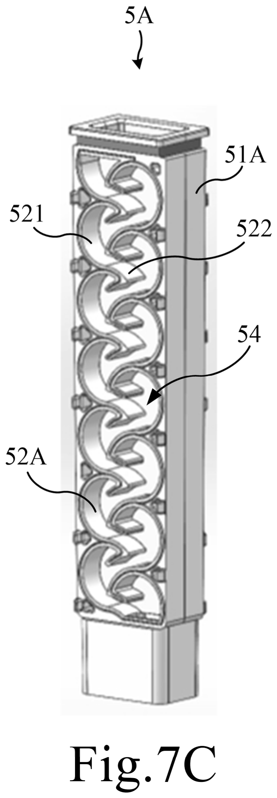

Figs. 7C to 7G illustrate cross-sectional views of filter channel devices according to several exemplary embodiments of the present invention.

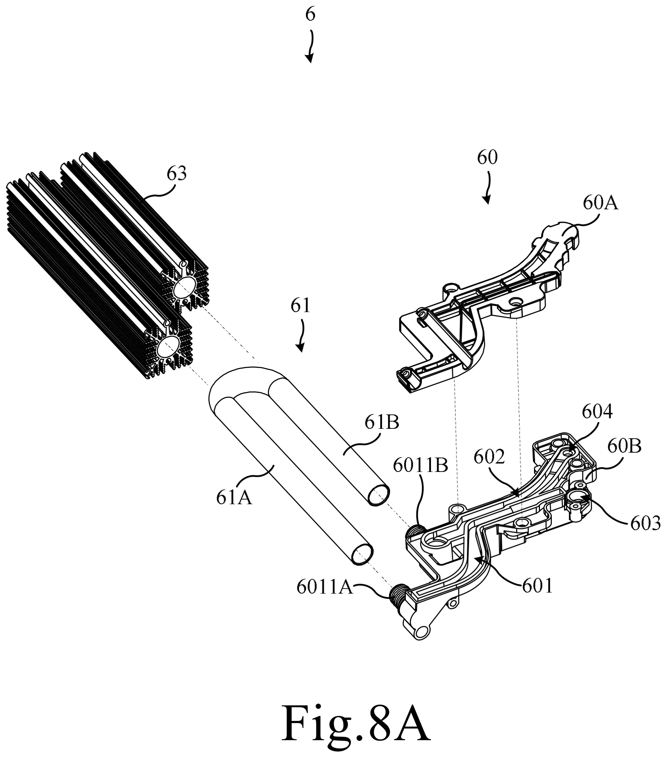

Fig. 8A is an exploded view of the condenser of Fig. 3.

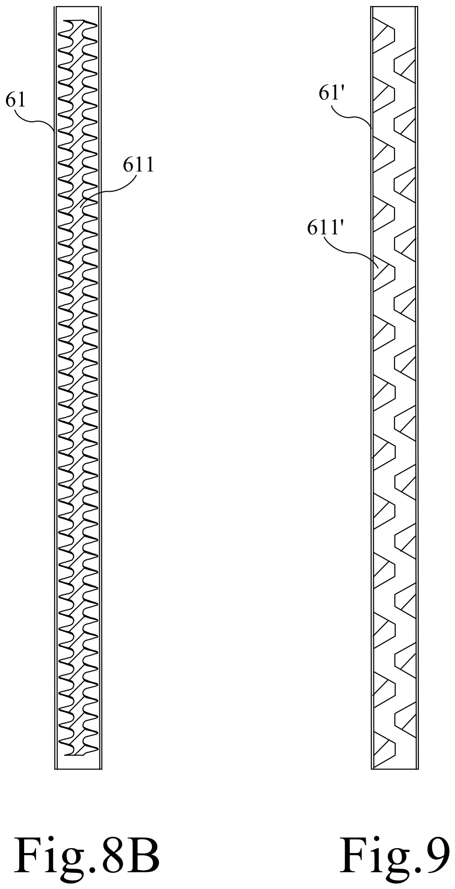

Fig. 8B is a cross-sectional view of a condensation tube of the condenser of Fig. 3.
Fig. 9 is a cross-sectional view of a condensation tube of a condenser according to an exemplary embodiment of the present invention.

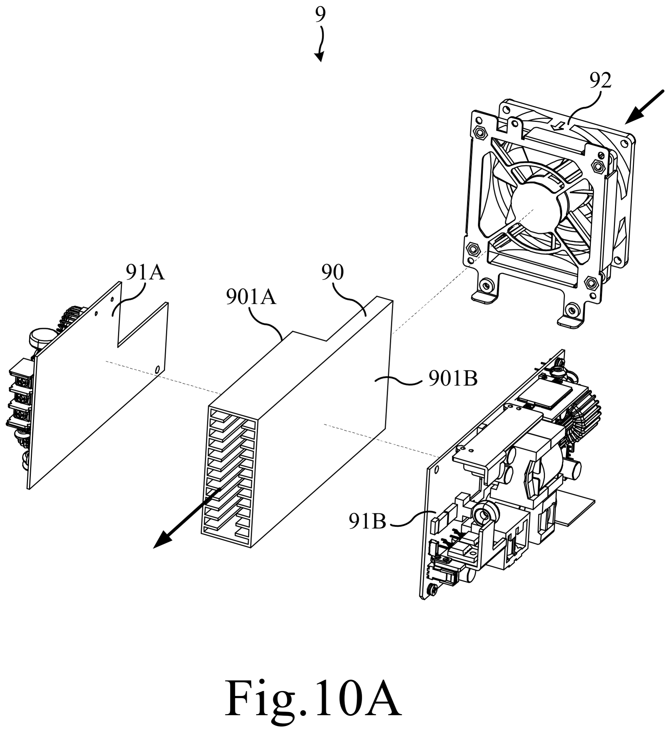

Fig. 10A is an exploded view of the power module of Fig. 1.

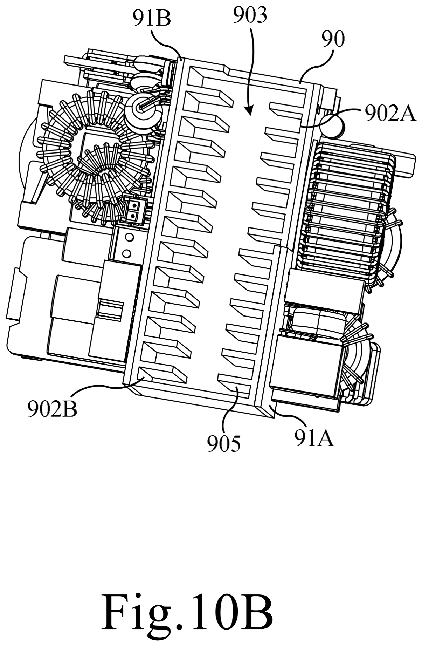

Fig. 10B is a schematic view of the base and circuit boards of the power module from another perspective.

The advantages, spirit, and features of the present invention will be set forth in detail in the following description of embodiments with reference to the accompanying drawings.

### DETAILED DESCRIPTION OF THE INVENTION

For the sake of the advantages, spirits and features of the present invention can be understood more easily and clearly, the detailed descriptions and discussions will be made later by way of the embodiments and with reference of the diagrams. It is worth noting that these embodiments are merely representative embodiments of the present invention, wherein the specific methods, devices, conditions, materials and the like are not limited to the embodiments of the present invention or corresponding embodiments. Moreover, the devices in the figures are only used to express their corresponding positions and are not drawing according to their actual proportion.

In the description of the present invention, it is to be understood that the orientations or positional relationships of the terms "longitudinal, lateral, upper, lower, front, rear, left, right, top, bottom, inner, outer" and the like are based on the orientation or positional relationship shown in the drawings. It is merely for the convenience of the description of the present invention and the description of the present invention, and is not intended to indicate or imply that the device or component referred to has a specific orientation, is constructed and operated in a specific orientation, and therefore cannot be understood as limitations of the invention.

In the description of this specification, the description with reference to the terms "a specific embodiment", "another specific embodiment" or "parts of specific embodiments" etc. means that the specific feature, structure, material or feature described in conjunction with the embodiment include in at least one embodiment of the present invention. In this specification, the schematic representations of the above-mentioned terms do not necessarily refer to the same embodiment. Moreover, the described specific features, structures, materials or characteristics can be combined in any one or more embodiments in a suitable manner.

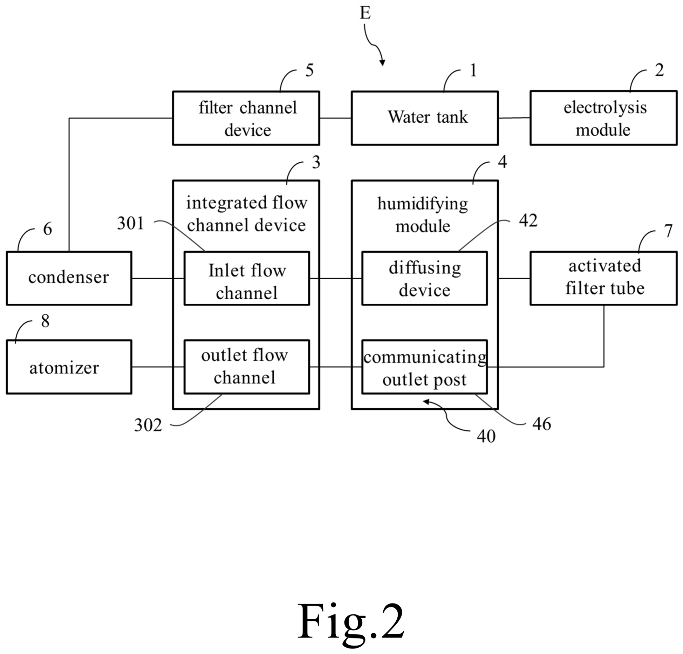

Please refer to Figs. 1 to 4B. Fig. 1 is a schematic structural diagram of a hydrogen generator E with a sound-proof shield according to an exemplary embodiment of the present invention. Fig. 2 is a functional block diagram of the hydrogen generator E with the sound-proof shield of Fig. 1. Fig. 3 is an exploded view of the hydrogen generator E with the sound-proof shield of Fig. 1. Fig. 4A is an exploded view of the water tank 1 of Fig. 1. Fig. 4B is a schematic structural diagram of the humidifying module 4 of Fig. 1. As shown in Figs. 1 to 4B, the hydrogen generator E with the sound-proof shield includes a water tank 1, an electrolysis module 2, an integrated flow channel device 3, a humidifying module 4, a filter channel device 5, and a condenser 6. The water tank 1 has an accommodation space 111 configured to contain electrolysis water. The electrolysis module 2 is disposed in the accommodation space 111 of the water tank 1 and is configured to receive the electrolysis water from the water tank 1, electrolyze the electrolysis water, and generate and output a gas comprising hydrogen into the water tank 1. The humidifying module 4 is disposed above the water tank 1 and is configured to receive and humidify the gas comprising hydrogen. The humidifying module 4 includes a humidifying chamber 40 and a gas delivery channel 41, and the humidifying chamber 40 and the gas delivery channel 41 are isolated from each other. The filter channel device 5 passes through the gas delivery channel 41 of the humidifying module 4 and is coupled to the water tank 1, and is configured to receive and filter the gas comprising hydrogen generated by the electrolysis module 2 and output the filtered gas comprising hydrogen. The condenser 6 is disposed above the humidifying module 4 and connected to the filter channel device 5. The condenser 6 is configured to receive and condense the gas comprising hydrogen output by the filter channel device 5. The integrated flow channel device 3 is disposed above the humidifying module 4 and located between the humidifying module 4 and the condenser 6. The integrated flow channel device 3 is connected to the condenser 6 and the humidifying module 4 and is configured to guide the gas comprising hydrogen output by the condenser 6 into the humidifying module 4. The hydrogen generator E with the sound-proof shield is a stack-type assembly, and the components are arranged from bottom to top as the water tank 1, the humidifying module 4, the integrated flow channel device 3, and the condenser 6.

In this embodiment, outer walls of the water tank 1 and the humidifying module 4 include a plurality of rib structures 13, and the plurality of rib structures 13 form a honeycomb structure. In practice, shapes formed by the rib structures 13 are not limited thereto. The plurality of rib structures 13 are configured to enhance structural strength of the water tank 1 and the humidifying module 4 so as to prevent deformation of the water tank 1 and the humidifying module 4 due to pressure differences generated by generation and flow of the gas comprising hydrogen.

In this embodiment, the water tank 1 may include a cover 10 and a tank body 11. The tank body 11 may define an accommodation space 111 configured to contain electrolysis water, and the cover 10 may cover the tank body 11 and the accommodation space 111. The electrolysis module 2 is disposed in the accommodation space 111 of the tank body 11; accordingly, the electrolysis module 2 is directly immersed in the water contained in the accommodation space 111 and directly draws water from the accommodation space 111 for electrolysis without piping connections. The electrolysis module 2 includes an electrolysis-module fixing plate 21, an electrolysis-module body 210, and an electrode plate assembly 20 disposed in the electrolysis-module body 210. For clarity in illustration, Fig. 2 only depicts portions of an anode plate 202 and a cathode plate 204 of the electrode plate assembly 20. In this embodiment, the electrode plate assembly 20 includes the anode plate 202, the cathode plate 204, and bipolar electrode plates located between them. The electrode plates are arranged in the electrolysis-module body 210 with intervals therebetween, so an electrode flow channel is formed between two adjacent electrode plates, and all of the electrode plates form a plurality of parallel electrode flow channels to electrolyze the water therein and generate hydrogen and oxygen, namely a gas comprising hydrogen. A top of the electrolysis-module body 210 has a plurality of upper openings respectively communicating the above electrode flow channels with an upper portion of the accommodation space 111; correspondingly, a bottom of the electrolysis-module body 210 also has a plurality of lower openings (not shown in Fig. 4A) communicating the above electrode flow channels with a lower portion of the accommodation space 111. Through the upper openings and the lower openings, the electrolysis-module body 210 receives water from the accommodation space 111 into the electrode flow channels for electrolysis and outputs the gas comprising hydrogen generated by electrolysis from the electrode flow channels into the accommodation space 111. Accordingly, in the hydrogen generator of the present invention, the housing of the electrolysis module is entirely located in the water tank, so that the electrolysis module is cooled by the water contained in the water tank. In practice, the hydrogen generator may include a cooling circulation system connected to the accommodation space of the water tank to perform cooling circulation on the water in the accommodation space. Meanwhile, no piping connection is required between the electrolysis module and the water tank for transporting water, thereby avoiding leakage of water or gas caused by deterioration of piping after long-term use.

The anode plate 202 and the cathode plate 204 may extend outward from the electrolysis-module body 210, as shown in Fig. 4A. Portions of the anode plate 202 and the cathode plate 204 that extend outward may be located in holes of the cover 10 of the water tank 1 and contact a power supply in the holes to receive electric power required for electrolysis. Please refer to Fig. 4C, which is the cover 10 of Fig. 4A from another perspective. As shown in Fig. 4C, a surface of the cover 10 facing the accommodation space 111 has through holes 1020 into which the anode plate 202 and the cathode plate 204 are inserted. The cover 10 further includes a first fixing portion 1022 toward the accommodation space 111, and the first fixing portion 1022 includes two annular fixing members respectively surrounding peripheries of the through holes 1020, as shown in Fig. 4C. In addition, the electrolysis-module body 210 in Fig. 4A has a second fixing portion 2108, and the second fixing portion 2108 includes two annular members respectively surrounding peripheries of positions where the anode plate 202 and the cathode plate 204 extend outward. Therefore, when the electrolysis module 2 is disposed in the water tank 1, the anode plate 202 and the cathode plate 204 are located in the through holes 1020, and the annular fixing members of the first fixing portion 1022 around the through holes 1020 engage with the annular members of the second fixing portion 2108 around the anode plate 202 or the cathode plate 204. Through engagement between the first fixing portion 1022 and the second fixing portion 2108, the electrolysis module 2 is engaged with the cover 10 and linked thereto, i.e., the electrolysis module 2 is suspended from the cover 10 within the accommodation space 111 of the water tank 1.

In practice, when the electrolysis module 2 electrolyzes water to generate the gas comprising hydrogen into the accommodation space 111 of the water tank 1, the gas comprising hydrogen first accumulates at an upper portion of the accommodation space 111 and is output upward to the filter channel device 5. During accumulation of the gas, pressure inside the water tank 1 increases and pushes the cover 10 outward, causing a slight bulging deformation. Because the electrolysis module 2 is suspended from and linked with the cover 10, regardless of a degree of upward bulging of the cover 10, engagement between the annular fixing members of the first fixing portion 1022 and the annular members of the second fixing portion 2108 keeps the electrolysis module 2 and its anode plate 202 and cathode plate 204 at a consistent relative position with respect to the cover 10. Likewise, when the electrolysis module 2 stops electrolyzing or when pressure inside the water tank 1 is released, the cover 10 returns to its original shape or position and, in linkage, keeps the electrolysis module 2 at the consistent relative position with the cover 10. Since the electrolysis module 2 moves in linkage with the cover 10 to maintain the relative position, no irreversible offset occurs between the electrolysis module 2 and the cover 10 after long-term use that could lead to water or gas leakage. In particular, portions of the anode plate 202 and the cathode plate 204 extending from the electrolysis-module body 210 are completely sealed within the through holes 1020 of the cover 10, thereby avoiding a risk that moisture enters the through holes 1020 and contacts the anode plate 202 and the cathode plate 204 due to positional deviation.

In this embodiment, the annular fixing members of the first fixing portion 1022 and the annular members of the second fixing portion 2108 are engaged with each other by hot-melt bonding. However, the present invention is not limited thereto. In practice, any fixing manner that can securely engage the two and seal, within holes of the upper cover, portions of the anode plate and the cathode plate extending from the electrolysis-module housing may be adopted by the hydrogen generator.

As shown in Fig. 4C, a surface of the cover 10 facing the accommodation space 111 is provided with a first positioning structure 1024, and a corresponding position on the electrolysis-module body 210 of the electrolysis module 2 is provided with a second positioning structure 2100. The first positioning structure 1024 and the second positioning structure 2100 are coupled to each other when the electrolysis module 2 is suspended from the cover 10 and maintain a freedom of vertical movement between the first positioning structure 1024 and the second positioning structure 2100. Specifically, the first positioning structure 1024 is a positioning hole, and the second positioning structure 2100 is a positioning post; when the electrolysis module 2 is suspended from the cover 10, the second positioning structure 2100 is received in the first positioning structure 1024 and is movable up and down. By virtue of the first positioning structure 1024 and the second positioning structure 2100, lateral offset between the cover 10 and the electrolysis module 2 can be suppressed when the cover 10 is deformed due to pressure accumulated in the water tank 1, thereby further suppressing water or gas leakage. In this embodiment, both of the two first positioning structures 1024 are positioning holes and both of the two second positioning structures 2100 are positioning posts; however, the present invention is not limited thereto, and the two first positioning structures may be positioning posts while the two second positioning structures may be positioning holes, or the first positioning structure and the second positioning structure may respectively include one positioning post and one positioning hole. In addition, positions of the first positioning structure 1024 and the second positioning structure 2100 in this embodiment are located at sides of the through holes 1020 of the cover 10 adjacent to the anode plate 202 and the cathode plate 204, and their number is two, but the present invention is not limited to the number and positions thereof and they may be set according to needs of a user or a designer.

On the other hand, please also refer to Figs. 4D and 4E. Fig. 4D is the tank body 11 of the water tank 1 of Fig. 4A from another perspective, and Fig. 4E is the electrolysis module 2 of Fig. 4A from another perspective. As shown in Figs. 4D and 4E, the tank body 11 of the water tank 1 has a plurality of third positioning structures 1100 at a bottom of the accommodation space 111, and the electrolysis-module body 210 forms a plurality of fourth positioning structures 2102 at a bottom thereof, the fourth positioning structures 2102 respectively corresponding to the third positioning structures 1100 on the tank body 11.

In this embodiment, when the electrolysis module 2 is disposed in the accommodation space 111 of the water tank 1 and suspended from the cover 10, third positioning structures 1100 of the tank body 11 of the water tank 1 are movably coupled to corresponding fourth positioning structures 2102 of the electrolysis-module body 210. Specifically, the third positioning structure 1100 may be a positioning post, and the fourth positioning structure 2102 may be a tubular positioning post; therefore, the third positioning structure 1100 can be received in the fourth positioning structure 2102. Because the third positioning structure 1100 and the fourth positioning structure 2102 extend vertically, the third positioning structure 1100 is movable up and down within the fourth positioning structure 2102 but is not laterally movable. When the electrolysis module 2 electrolyzes water to generate the gas comprising hydrogen accumulating in the water tank 1, the cover 10 is slightly bulged by pressure from the accommodation space 111 and thereby moves the electrolysis module 2 suspended from the cover 10; conversely, when the electrolysis module 2 stops electrolyzing or when pressure in the water tank 1 is released, pressure accumulated in the water tank 1 disappears, the cover 10 returns to its original state, and the suspended electrolysis module 2 is moved accordingly. By virtue of the third positioning structure 1100 of the tank body 11 and the fourth positioning structure 2102 of the electrolysis-module body 210, a direction of relative movement between the electrolysis module 2 and the tank body 11 is limited to vertical movement only. Since the electrolysis module 2 is suspended from the cover 10 and is not fixed to the tank body 11 by screws or the like, deformation of the cover 10 under pressure could tilt the electrolysis module 2 in the accommodation space 111. Accordingly, by allowing only vertical relative movement between the tank body 11 and the electrolysis module 2 through the third positioning structure 1100 and the fourth positioning structure 2102, tilting of the electrolysis module 2 in the water tank 1 that could cause water or gas leakage is prevented.

In this embodiment, numbers of the third positioning structures 1100 and the fourth positioning structures 2102 are three, and they are disposed at positions shown in Figs. 4D and 4E. In practice, the present invention does not limit numbers and positions of the third positioning structures and the fourth positioning structures and they may be set according to needs of a user or a designer. However, to effectively restrict the entire electrolysis module to move only in a vertical direction, the third positioning structures and the fourth positioning structures may be at least two and distributed at different portions on a bottom of the tank body and on the electrolysis-module housing, thereby effectively preventing the electrolysis module from tipping laterally in the water tank. In addition, it is noted that coupling between the first positioning structures 1024 of the cover 10 and the second positioning structures 2100 of the electrolysis-module body 210 not only positions a relative relationship between the cover 10 and the electrolysis module 2, but also allows the electrolysis module 2 to move up and down with deformation and restoration of the cover 10.

Furthermore, as shown in Fig. 4E, a bottom of the electrolysis-module body 210 has a plurality of lower openings 2109, which, as described above, communicate electrode flow channels formed by a plurality of electrodes in the electrolysis-module body 210 with a lower portion of the accommodation space 111. Because the electrolysis module 2 is disposed in the accommodation space 111 of the water tank 1 and can be immersed in water, water in the water tank 1 can be directly received through the lower openings 2109 for electrolysis to generate the gas comprising hydrogen. Thus, the electrolysis module 2 of the hydrogen generator 1 can receive water without piping, thereby avoiding leakage of water or gas caused by deterioration or even detachment of piping after long-term use.

In summary, in this embodiment the electrolysis module is suspended in the water tank and is connected to, and moves in linkage with, the upper cover of the water tank. When the electrolysis module electrolyzes water to generate a gas comprising hydrogen and pressure in the water tank rises, bulging deformation of the upper cover caused by the pressure drives the electrolysis module to move while maintaining its relative position with the upper cover. Accordingly, electrode plates extending from the electrolysis module remain sealed within the through holes of the upper cover, and no irreversible offset occurs after long-term use that would lead to water or gas leakage. In addition, positioning structures on the tank body, the upper cover, and the electrolysis-module housing keep the electrolysis module from laterally shifting or tilting in the water tank, thereby further avoiding leakage risk.

Furthermore, the electrolysis-module fixing plate 21 further includes a partition plate 211. The partition plate 211 is configured to fix the electrolysis module 2 in the water tank 1 and to divide the water tank 1 into an upper layer and a lower layer, such that electrolysis water mainly resides in the lower layer and a gas comprising hydrogen generated by electrolysis mainly resides in the upper layer. To maintain communication between the upper and lower layers, the partition plate 211 has a plurality of flow-through holes 2110 communicating the upper and lower layers. The electrolysis-module fixing plate 21 may be formed integrally. In addition, those skilled in the art may design a shape of the partition plate 211 as needed to provide installation space for other components.

Please also refer to Figs. 3, 4F, 4G, 5, and 6B. Fig. 4F is an assembled view of the integrated flow channel device 3, the filter channel device 5, and the condenser 6 of Fig. 1. Fig. 4G is an exploded view of the integrated flow channel device 3, the filter channel device 5, and the condenser 6 of Fig. 1. Fig. 5 is a top view of the hydrogen generator E with the sound-proof shield of Fig. 1. Fig. 6B is a cross-sectional view taken along line B-B of Fig. 5. As shown in Figs. 3, 4F, and 4G, in this embodiment the humidifying module 4 is vertically stacked above the water tank 1, the integrated flow channel device 3 is vertically stacked above the humidifying module 4, the condenser 6 is fixed above the integrated flow channel device 3, and the filter channel device 5 penetrates the humidifying module 4 and the integrated flow channel device 3 and is directly connected to the condenser 6. The gas delivery channel 41 of the humidifying module 4 is a through hole extending vertically from a bottom of the humidifying module 4 to a top thereof. Furthermore, the integrated flow channel device 3 includes an opening 303 corresponding to the gas delivery channel 41 of the humidifying module 4, and a bottom of the filter channel device 5 passes through both the opening 303 of the integrated flow channel device 3 and the gas delivery channel 41 of the humidifying module 4 to connect to the water tank 1. Therefore, a gas comprising hydrogen generated in the water tank 1 can directly flow in sequence to the filter channel device 5 and the condenser 6 without flowing into interiors of the integrated flow channel device 3 and the humidifying module 4. In addition, a length of the filter channel device 5 may be greater than a combined length of the opening 303 and the gas delivery channel 41. When the hydrogen generator E with the sound-proof shield is assembled, a top of the filter channel device 5 may protrude above the integrated flow channel device 3. Thus, a user can grasp the top and directly pull out the filter channel device 5 for replacement or cleaning without disassembling other components or devices, thereby improving convenience and installation efficiency.

As shown in Fig. 4G, in this embodiment the integrated flow channel device 3 includes a fixing structure 304, and the condenser 6 includes a matching structure 605 corresponding to and matching the fixing structure 304. The matching structure 605 of the condenser 6 can be connected to the fixing structure 304 by locking, thereby fixing the condenser 6 on the integrated flow channel device 3. Since the condenser 6 is located at a topmost portion of the hydrogen generator and the filter channel device 5 is a component that can be pulled out and detached, after the filter channel device 5 is extracted the user can directly detach and clean the condenser 6 without disassembling other components or devices, thereby improving convenience and installation efficiency.

Please also refer to Figs. 1, 3, 7A, and 7B. Fig. 7A is an exploded view of the filter channel device 5 of Fig. 3. Fig. 7B is a cross-sectional view of the filter channel device 5 of Fig. 3. As shown in Figs. 3, 7A, and 7B, in this embodiment the filter channel device 5 includes a channel housing 51 having a cavity, and dimensions of the channel housing 51 correspond to dimensions of the gas delivery channel 41 of the humidifying module 4 and an opening 303 of the integrated flow channel device 3. The channel housing 51 has a first end 511 and a second end 512, and an upper cover 501 is provided at the first end 511. The first end 511 protrudes above the integrated flow channel device 3 and is directly connected to the condenser 6, and the second end 512 is connected to the cover 10 of the water tank 1. The second end 512 of the channel housing 51 includes a mounting structure 513, and the cover 10 of the water tank 1 includes an assembling structure 101 matching the mounting structure 513. When the hydrogen generator E with the sound-proof shield is assembled, the mounting structure 513 of the channel housing 51 can be snap-fitted and tightly fitted to the assembling structure 101 of the water tank 1. Therefore, the gas comprising hydrogen generated by the electrolysis module 2 can directly flow from the water tank 1 to the filter channel device 5 without leaking outside the water tank 1.

Furthermore, the filter channel device 5 has an opening 5011, and the mounting structure 513 and the assembling structure 101 of the water tank 1 respectively have openings. That is, the opening 5011 of the filter channel device 5, the cavity of the channel housing 51, the opening of the mounting structure 513, the opening of the assembling structure 101, and the accommodation space 111 of the water tank 1 are in communication with one another. Therefore, after the electrolysis module 2 electrolyzes the electrolysis water and generates the gas comprising hydrogen, the gas comprising hydrogen flows from the accommodation space 111 of the water tank 1 through the openings of the assembling structure 101 and the mounting structure 513 to the filter channel device 5. Then, after the filter channel device 5 filters the gas comprising hydrogen, the gas comprising hydrogen flows through the opening 5011 to the condenser 6.

In this embodiment, the filter channel device 5 further includes a filtering member 52 disposed in a cavity of the channel housing 51 and configured to filter alkaline substances, impurities, and electrolytes from the gas comprising hydrogen generated by the electrolysis module 2. In practice, the filtering member 52 may be, but is not limited to, filter cotton. As shown in Fig. 7B, the filter channel device 5 further includes two mesh metal elements 53 respectively disposed and fixed at a first end 511 and a second end 512 of the channel housing 51, and the filtering member 52 is located between the two mesh metal elements 53. In practice, when the gas comprising hydrogen generated by the electrolysis module 2 flows from the water tank 1 through the filter channel device 5, the filtering member 52 in the channel housing 51 may move with the gas flow. Accordingly, the mesh metal elements 53 limit a range of movement of the filtering member 52. In addition, the mesh metal elements 53 are structures having voids, so the gas comprising hydrogen can pass through the mesh metal elements 53 without being blocked. Furthermore, the mesh metal elements 53 may also have a filtering function to filter alkaline substances, impurities, and electrolytes from the gas comprising hydrogen. In another exemplary embodiment, the filter channel device may include only the mesh metal elements without the filter cotton.

The hydrogen generator E with the sound-proof shield further includes a conductive member (e.g., a metal rod). The conductive member extends downward from a bottom of the filter channel device 5 to the electrolysis water of the water tank 1. In one embodiment, one end of the conductive member is disposed at a bottom position of the filter channel device 5, and the other end extends into the water tank 1.

Please also refer to Figs. 3, 5, 8A, and 8B. Fig. 8A is an exploded view of the condenser 6 of Fig. 3. Fig. 8B is a cross-sectional view of a condensation tube 61 of the condenser 6 of Fig. 3. As shown in Fig. 8A, in this embodiment the condenser 6 includes a seat 60 and a condensation tube 61. The seat 60 includes an upper seat 60A and a lower seat 60B that are matched and assembled with each other, and the lower seat 60B includes a first flow channel 601 and a second flow channel 602, which are isolated from each other. Furthermore, the lower seat 60B has a condensation inlet 603 and a condensation outlet 604. The condensation inlet 603 communicates with the first flow channel 601 and directly communicates with the opening 5011 of the filter channel device 5 (see Figs. 4G and 7A), and the condensation outlet 604 communicates with the second flow channel 602 and an inlet flow channel 301 of the integrated flow channel device 3 (see Fig. 6B). In practice, the upper seat 60A may also include two flow channels corresponding to and matching the first flow channel 601 and the second flow channel 602, so that the seat 60 of the condenser 6 forms two independent and separated flow channels. The form of the upper seat is not limited thereto; the upper seat may also be a flat plate without flow channels, and the gas comprising hydrogen then flows only in the first flow channel 601 and the second flow channel 602 of the lower seat 60B.

As shown in Figs. 3 and 5, in this embodiment the condenser 6 is generally L-shaped. When the condenser 6 is assembled, the seat 60 of the condenser 6 is mounted on a top surface of the integrated flow channel device 3, and the condensation tube 61 of the condenser 6 is suspended at a side of the integrated flow channel device 3. Furthermore, a length of the seat 60 of the condenser 6 accounts for more than two thirds of a side-edge length of the hydrogen generator E. Therefore, after the matching structure 605 of the condenser 6 is locked to the fixing structure 304 of the integrated flow channel device 3 (see Fig. 4G), the condenser 6 can be fixed more securely on the integrated flow channel device 3 and is able to support the condensation tube 61. In practice, the length of the seat 60 of the condenser 6 is not limited thereto; the length of the seat 60 may also account for more than one half of the side-edge length of the hydrogen generator E.

In this embodiment, the condensation tube 61 of the condenser 6 includes a first condensation tube 61A and a second condensation tube 61B, and the lower seat 60B includes a first connection hole 6011A and a second connection hole 6011B with threads. One end of the first condensation tube 61A is connected to the first connection hole 6011A to communicate with the first flow channel 601, and one end of the second condensation tube 61B is connected to the second connection hole 6011B to communicate with the second flow channel 602. Free ends of the first condensation tube 61A and the second condensation tube 61B not connected to the seat 60 may be mutually connected by a connecting tube, so that the condensation tube 61 forms a single channel. End portions of the first condensation tube 61A and the second condensation tube 61B may respectively include internal threads matching the first connection hole 6011A and the second connection hole 6011B. After assembly of the condenser 6, the condensation inlet 603, the first flow channel 601, the first condensation tube 61A, the second condensation tube 61B, the second flow channel 602, and the condensation outlet 604 form a single-path condensation flow channel. In this embodiment, the first condensation tube 61A and the second condensation tube 61B are arranged horizontally side by side to reduce a volume and a height of the hydrogen generator E with the sound-proof shield. In practice, an arrangement of the first condensation tube and the second condensation tube may be determined according to design or requirements. Furthermore, the condenser may include more than two condensation tubes, and a single channel may be formed by connecting the plurality of condensation tubes through a plurality of connecting tubes.

In addition, the condenser 6 further includes a heat-dissipation element 63 that contacts and is disposed outside the first condensation tube 61A and the second condensation tube 61B. In this embodiment, the heat-dissipation element 63 is an aluminum extrusion, and the aluminum extrusion has holes sized to correspond to the condensation tubes for the condensation tubes to pass through and for heat to be dissipated from the condensation tubes. In practice, because aluminum has a good thermal conductivity, when the gas comprising hydrogen flows through the condensation tube 61, the aluminum extrusion absorbs heat energy from the gas comprising hydrogen by heat conduction and exchanges heat with ambient air, so that moisture in the gas comprising hydrogen forms condensate, thereby improving condensation efficiency.

As shown in Fig. 8B, the condenser 6 further includes a plurality of helical structures 611 respectively disposed in the first condensation tube 61A and the second condensation tube 61B of the condensation tube 61 (Fig. 8B schematically shows only one condensation tube 61). In practice, the helical structure 611 may be an I-shaped helical post disposed in the condensation tube 61 to extend path lengths inside the first condensation tube 61A and the second condensation tube 61B of the condensation tube 61, namely to increase a length of the condensation flow channel. Therefore, when the gas comprising hydrogen passes through the condensation tube 61 of the condenser 6, the gas comprising hydrogen flows along the helical structures 611 through the condensation tube 61, and a residence time of the gas comprising hydrogen in the condensation tube 61 is extended, thereby improving condensation efficiency.

In this embodiment, the hydrogen generator E with the sound-proof shield includes a valve 32 disposed on the integrated flow channel device 3 and communicating the condenser 6 and the humidifying module 4, and the integrated flow channel device 3 includes an inlet flow channel 301. The valve 32 includes a first valve interface 321 and a second valve interface 322. The first valve interface 321 communicates with a condensation outlet 604 of the condenser 6, and the second valve interface 322 communicates with the inlet flow channel 301 of the integrated flow channel device 3. When the condenser 6 outputs the condensed gas comprising hydrogen, the gas comprising hydrogen sequentially flows through the condensation outlet 604, the first valve interface 321, the second valve interface 322, the inlet flow channel 301, and then to the humidifying module 4.

Please also refer to Figs. 4B, 4B-1, 4B-2, 4B-3, and 4B-4. As shown in Figs. 4B-1 to 4B-3, in this embodiment the hydrogen generator E with the sound-proof shield includes a diffusing device 42 and a sound-proof shield 43. The diffusing device 42 is disposed in the humidifying chamber 40 and located at a bottom of the humidifying module 4. The sound-proof shield 43 is disposed in the humidifying chamber 40 and stacked above the diffusing device 42. The sound-proof shield 43 has a sound-proof cavity 430 configured to contain supplement water and the diffusing device 42. In practice, a bottom of the sound-proof shield 43 has an opening whose shape can correspond to a shape of the diffusing device 42. A bottom of the humidifying module 4 includes a locking structure 401, the diffusing device 42 includes a mounting hole 420, and the sound-proof shield 43 includes a locking sleeve 4310. The mounting hole 420 of the diffusing device 42 can pass through the locking structure 401, the locking sleeve 4310 of the sound-proof shield 43 is then sleeved on the locking structure 401, and finally a screw can lock the locking sleeve 4310 to the locking structure 401, so that the diffusing device 42 and the sound-proof shield 43 are fixed in the humidifying chamber 40 and the sound-proof shield 43 covers the diffusing device 42. It is noted that positions and installation manners of the diffusing device 42 and the sound-proof shield 43 are not limited thereto.

In this embodiment, the sound-proof shield 43 includes a connecting tube 431 isolated from the sound-proof cavity 430, and the connecting tube 431 is in gas communication with the inlet flow channel 301 of the integrated flow channel device 3 and the diffusing device 42. As shown in Figs. 4B-2 to 4B-4, the connecting tube 431 has an inlet port 4311 and an outlet port 4312, and the diffusing device 42 includes an inlet 422. When the integrated flow channel device 3 and the humidifying module 4 are assembled, the inlet port 4311 communicates with the inlet flow channel 301 and the outlet port 4312 communicates with the inlet 422. When the inlet flow channel 301 outputs the condensed gas comprising hydrogen, the gas comprising hydrogen first flows through the connecting tube 431 and to the diffusing device 42, and does not directly enter the humidifying chamber 40.

Furthermore, the diffusing device 42 may include a plurality of micro-holes 421. Since the diffusing device 42 is accommodated in the sound-proof cavity 430, the micro-holes 421 of the diffusing device 42 communicate with the sound-proof cavity 430. Therefore, after the condensed gas comprising hydrogen flows through the connecting tube 431 and to the diffusing device 42, the gas comprising hydrogen passes through the micro-holes 421 of the diffusing device 42 and enters the supplement water in the sound-proof cavity 430 to form microbubbles, so that the gas comprising hydrogen is sufficiently filtered and humidified by the supplement water in the sound-proof cavity 430. In addition, in this embodimenta top of the sound-proof shield 43 has a plurality of gas outlets 432. After the gas comprising hydrogen flows through the micro-holes 421 of the diffusing device 42 into the sound-proof cavity 430, the gas comprising hydrogen flows from a bottom of the sound-proof shield 43 to a top thereof and flows to the humidifying chamber 40 through the gas outlets 432.

As shown in Figs. 4B-1 to 4B-4, in this embodiment the hydrogen generator E with the sound-proof shield further includes filter sheets 435, and the integrated flow channel device 3 further includes securing structures 33. The filter sheets 435 are disposed outside the sound-proof shield 43 and configured to cover the gas outlets 432, and the securing structures 33 are configured to contact and abut against the filter sheets 435 so that the filter sheets 435 are attached to the sound-proof shield 43 and cover the gas outlets 432. In practice, a top of the sound-proof shield 43 has groove structures 433, and the gas outlets 432 are located corresponding to the groove structures 433. A material of the filter sheet 435 may be a porous sintered plastic, for example a polypropylene (PP) sintered sheet, but is not limited thereto. A shape of the filter sheet 435 may correspond to a shape of the groove structure 433, and holes of the filter sheet 435 are smaller than the gas outlets 432. That is, the filter sheet 435 can be disposed in the groove structure 433. The securing structures 33 extend outward from a surface of the integrated flow channel device 3 facing the humidifying module 4, and positions of the securing structures 33 correspond to positions of the groove structures 433. When the integrated flow channel device 3 and the humidifying module 4 are assembled, the securing structures 33 abut against the filter sheets 435 to retain the filter sheets 435 in the groove structures 433. Therefore, the gas comprising hydrogen located in the sound-proof cavity 430 sequentially passes through the gas outlets 432 and the filter sheets 435 and flows to the humidifying chamber 40. It is noted that, in this embodiment, the hydrogen generator E with the sound-proof shield includes four filter sheets 435, the sound-proof shield 43 includes four groove structures 433, and the integrated flow channel device 3 includes four securing structures 33. In practice, numbers of the filter sheets, the groove structures, and the securing structures may be determined according to design.

As shown in Fig. 4B-4, in this embodiment an outer wall of the sound-proof shield 43 and an inner wall of the humidifying module 4 define a gap G, and a water level of the supplement water disposed in the humidifying chamber 40 is lower than a mounting position of the filter sheets 435. In practice, when the gas comprising hydrogen flows from the supplement water to the humidifying chamber 40, disturbance and vibration may occur on a water surface and sound may be generated. When the gas comprising hydrogen flows through the micro-holes 421 of the diffusing device 42 into the sound-proof cavity 430 and reaches the water surface of the supplement water in the sound-proof cavity 430, disturbance of the water surface is transmitted only to the sound-proof shield 43 and not to the inner wall of the humidifying module 4, thereby confining sound generated by vibration within the sound-proof shield 43 to achieve a sound-proofing effect and improve user experience.

In the hydrogen generator E with the sound-proof shield of the present invention, the water tank 1, the humidifying module 4, the filter channel device 5, the condenser 6, and the integrated flow channel device 3 are at an equal electrical potential, and the hydrogen generator E with the sound-proof shield may further include a housing (not shown) accommodating the above components, and the water tank 1, the humidifying module 4, the filter channel device 5, the condenser 6, and the integrated flow channel device 3 are electrically connected to the housing.

Please also refer to Figs. 3, 4C, 4D, and 6C. Fig. 6C is a cross-sectional view taken along line C-C of Fig. 5. In this embodiment, the hydrogen generator E with the sound-proof shield further includes an activated carbon tube or an activated filter tube 7 in gas communication with the humidifying module 4 and the integrated flow channel device 3. As shown, a cover 10 of the water tank 1 includes a cover channel 103, and a tank body 11 includes a tank-body channel 113. The cover channel 103 and the tank-body channel 113 correspond to each other; the cover channel 103 is isolated from an internal space of the cover 10, and the tank-body channel 113 is isolated from the accommodation space 111. A bottom of the humidifying module 4 has a mounting interface 44 and includes a communicating inlet post 45 and a communicating outlet post 46. The mounting interface 44, the communicating outlet post 46, the cover channel 103, and the tank-body channel 113 correspond to and communicate with one another. The communicating inlet post 45 communicates with the humidifying chamber 40 and includes an inlet hole 451, and the communicating inlet post 45 is not directly in communication with the communicating outlet post 46. The activated filter tube 7 includes a housing 70, and a top of the activated filter tube 7 can penetrate the tank-body channel 113 and the cover channel 103 and be snap-fitted to the mounting interface 44 to connect to the humidifying module 4. Furthermore, the communicating inlet post 45 is higher than a water surface of the supplement water in the humidifying chamber 40. When the activated filter tube 7 is mounted on the humidifying module 4, a top of the housing 70 abuts a bottom of the humidifying module 4, whereby a cavity 48 is formed between the housing 70 of the activated filter tube 7 and the bottom of the humidifying module 4, and the humidifying chamber 40, the inlet hole 451 of the communicating inlet post 45, and the cavity 48 are in communication with one another. In addition, the activated filter tube 7 filters the gas comprising hydrogen in the humidifying module 4. A bottom of the activated filter tube 7 includes an inlet 71 and a top thereof includes an outlet 72. The inlet 71 communicates with the cavity 48, and the outlet 72 communicates with the mounting interface 44 and the communicating outlet post 46. After the humidifying module 4 receives and humidifies the gas comprising hydrogen condensed by the condenser 6, the humidified gas comprising hydrogen flows from the inlet hole 451 of the communicating inlet post 45 to the cavity 48, passes through the inlet 71 into the activated filter tube 7 to filter impurities in the humidified gas comprising hydrogen. Furthermore, the integrated flow channel device 3 further includes an outlet flow channel 302 communicating with the communicating outlet post 46 of the humidifying module 4. Accordingly, the gas comprising hydrogen filtered by the activated filter tube 7 flows from the outlet 72 sequentially through the mounting interface 44, the communicating outlet post 46, and the outlet flow channel 302 to output the gas comprising hydrogen from the humidifying module 4.

In addition, the hydrogen generator E with the sound-proof shield further includes an atomizer 8 coupled to the outlet flow channel 302 of the integrated flow channel device 3 to receive the gas comprising hydrogen, and selectively generating an atomized gas to be mixed with the gas comprising hydrogen to form a health-care gas. The atomizer 8 may generate an atomized gas and mix it with the gas comprising hydrogen to form the health-care gas, wherein the atomized gas may be selected from the group consisting of water vapor, atomized medicinal solution, and volatile essential oil, or a combination thereof. In one exemplary embodiment, the atomizer 8 includes an oscillator that atomizes water, an atomized medicinal solution, or a volatile essential oil added to the atomizer 8 by oscillation to produce the atomized gas, and then mixes the mixed gas with the atomized gas to form the health-care gas. The atomizer 8 can be selectively turned on or off according to user needs to provide the user with the health-care gas of the mixed atomized gas, or to provide only the mixed gas (i.e., hydrogen diluted with secondary oxygen) for inhalation.

Please also refer to Figs. 3 and 6A to 6D. Fig. 6D is a simplified schematic diagram of a gas flow of the hydrogen generator E with the sound-proof shield according to an exemplary embodiment of the present invention, and a flow of the gas comprising hydrogen is indicated by arrows. During operation of the hydrogen generator E with the sound-proof shield, the gas comprising hydrogen generated by electrolysis of the electrolysis water by the electrolysis module 2 in the water tank 1 first flows from the accommodation space 111 of the water tank 1 through the filter channel device 5 and an opening 5011 of the filter channel device 5, and then flows into the condenser 6 from the condensation inlet 603. It is noted that while the condensation tube 61 condenses moisture in the gas comprising hydrogen, condensate generated by condensation also carries away residual electrolytes in the gas comprising hydrogen, so that the condenser 6 also has a filtering function. Next, the gas comprising hydrogen sequentially passes through the condensation outlet 604 of the condenser 6, the inlet flow channel 301 of the integrated flow channel device 3, and the micro-holes of the diffusing device 42 of the humidifying module 4, and then enters the humidifying chamber 40 of the humidifying module 4 to be humidified. Thereafter, the gas comprising hydrogen sequentially passes from the humidifying chamber 40 through the outlet flow channel 302 of the integrated flow channel device 3 and the activated filter tube 7, and then enters the atomizer 8. Finally, the atomizer 8 can be selectively turned on or off according to user needs to provide the user with the gas comprising hydrogen or the health-care gas of the mixed atomized gas for inhalation.

Please refer to Figs. 4B, 4B-4, 6B, and 6E. Fig. 6E is a simplified schematic diagram of a supplement-water flow of the hydrogen generator E with the sound-proof shield according to an exemplary embodiment of the present invention. As shown in Figs. 4B, 4B-4, and 6B, the integrated flow channel device 3 further includes a supplement water flow channel 306 and a supplement water port 307, and the sound-proof shield 43 includes a supplement water passage 434 communicating the supplement water flow channel 306 and the sound-proof cavity 430. The humidifying module 4 further includes a backflush tube 47, the valve 32 includes a third valve interface 323, and the backflush tube 47 is connected to the third valve interface 323. The hydrogen generator E with the sound-proof shield further includes a supplement water pump 308 disposed outside the water tank 1 and connected to the backflush tube 47. A supplement-water flow direction is indicated by arrows in Fig. 6E. During replenishment, supplement water is added from the supplement water port 307 of the integrated flow channel device 3, sequentially flows through the supplement water flow channel 306 of the integrated flow channel device 3 and the supplement water passage 434 of the sound-proof shield 43, and then flows into the sound-proof cavity 430 of the sound-proof shield 43. It is noted that a side of the sound-proof shield 43 may include holes to communicate the sound-proof cavity 430 with the humidifying chamber 40. Thereafter, the supplement water in the humidifying chamber 40 is delivered directly to the condenser 6 by the supplement water pump 308 disposed outside the water tank 1, and finally flows from the condenser 6 through the filter channel device 5 back to the water tank 1. It is noted that when the supplement water is backflushed from the condenser 6 to the water tank 1, the supplement water also backflushes alkaline substances and electrolytes remaining on the condenser 6 and the filter channel device 5 into the water tank 1. The first valve interface 321 of the valve 32 selectively communicates with the second valve interface 322 or the third valve interface 323 according to an operating state. During normal operation of the hydrogen generator, the first valve interface 321 communicates with the second valve interface 322 to guide the condensed gas comprising hydrogen from the condenser 6 to the diffusing device 42 for humidification; during replenishment, the first valve interface 321 communicates with the third valve interface 323 to deliver the supplement water through the backflush tube 47 to the condenser 6 and backflush the supplement water to the water tank 1.

The filter channel device of the hydrogen generator with the sound-proof shield is not limited to the forms of the foregoing exemplary embodiment and may take other forms. Please refer to Figs. 7C to 7G, which illustrate cross-sectional views of filter channel devices according to several exemplary embodiments of the present invention. As shown in Fig. 7C, a difference of the filter channel device 5A of this embodiment from the foregoing embodiment is that a filtering member 52A of the filter channel device 5A includes a plurality of baffle structures, and each baffle structure includes an arcuate portion 521 and a hooked portion 522 connected to the arcuate portion 521. The arcuate portion 521 extends upward, and the hooked portion 522 extends downward from a top of the arcuate portion 521. The baffle structures are staggered disposed in a channel housing 51A to form a filtering flow channel 54. In practice, the arcuate portion 521 may extend upward along an inner wall of the filter channel device 5A, and the hooked portion 522 may extend from the top of the arcuate portion 521 toward a lower-right direction and then toward a lower-left direction. The baffle structures may protrude in a staggered manner from opposite inner walls of the channel housing 51A into a cavity and may be integrally formed with the channel housing 51A. The filtering flow channel 54 may be an S-shaped flow channel. When the gas comprising hydrogen generated by the water tank 1 flows through the filtering flow channel 54 of the filter channel device 5A, the hooked portions 522 of the baffle structures block alkaline substances, impurities, and electrolytes so that the alkaline substances, impurities, and electrolytes adhere to and remain on the baffle structures of the filter channel device 5A, thereby achieving a filtering effect. Likewise, when the hydrogen generator with the sound-proof shield performs replenishment, the supplement water flows through the filtering flow channel 54 of the filter channel device 5A, and the supplement water backflushes the alkaline substances, impurities, and electrolytes remaining on the baffle structures back to the water tank. The baffle structures are not limited to the form shown in Fig. 7C and may take the forms shown in the filter channel devices 5B, 5C, 5D, and 5E of Figs. 7D to 7G. In practice, the hooked portion of the baffle structure may extend only toward the lower-right direction or the lower-left direction.

The condenser of the hydrogen generator with the sound-proof shield of the present invention is not limited to the forms of the foregoing exemplary embodiment and may take other forms. Please refer to Fig. 9, which illustrates a cross-sectional view of a condensation tube 61' of a condenser according to one exemplary embodiment of the present invention. As shown in Fig. 9, an inner wall of the condensation tube 61' is provided with retardation structures 611'. In practice, an inner surface of the condensation tube 61' may have a plurality of protrusions to form the retardation structures 611' so as to increase a path length of a condensation flow channel. The protrusions may also be formed as an internal thread structure on the inner surface of the condensation tube 61'. In another exemplary embodiment, the retardation structures are not limited to surface protrusions and may include other structures capable of retarding a liquid flow rate, such as a mesh structure. In addition, in one exemplary embodiment a heat-dissipation element of the condenser is a plurality of heat-dissipation fins. The heat-dissipation fins have a plurality of holes through which the condensation tube can extend. In practice, the heat-dissipation fins may be a two-piece assembly structure or a three-dimensional wavy structure to increase a heat-dissipation surface area per unit volume. The heat-dissipation fins may also be configured without holes and dissipate heat by surrounding the condensation tube. Furthermore, the plurality of heat-dissipation fins may be arranged at fixed intervals.

Please also refer to Figs. 1, 3, 10A, and 10B. Fig. 10A is an exploded view of a power module 9 of Fig. 1. Fig. 10B is a schematic view of a base 90 and circuit boards of the power module 9 from another perspective. In this embodiment, the power module 9 is disposed above the water tank 1 and electrically connected to the electrolysis module 2, and is configured to supply electric energy to the electrolysis module 2 so that the electrolysis module 2 electrolyzes the electrolysis water. As shown in Figs. 10A and 10B, the power module 9 includes a base 90 in a cuboid shape, a first circuit board 91A, and a second circuit board 92B. The base 90 includes a heat-dissipation channel 903 and has an outer surface and an inner surface, and the inner surface forms the heat-dissipation channel 903. Furthermore, the outer surface includes a first outer surface 901A and a second outer surface 901B opposite to each other, and the heat-dissipation channel 903 is located between the first outer surface 901A and the second outer surface 901B. The first circuit board 91A and the second circuit board 92B are respectively disposed on the first outer surface 901A and the second outer surface 901B of the base 90. In practice, a cover 10 of the water tank 1 may further include a mounting structure, and the power module 9 may further include a bracket fixable to the mounting structure, and the base 90 is connected to the bracket.

The power module 9 further includes a fan 92 disposed at one end of the base 90, and the base 90 includes a plurality of heat-dissipation fins 905. An inner surface of the base 90 includes a first inner surface 902A and a second inner surface 902B opposite to each other, and the heat-dissipation fins 905 respectively extend inward from the first inner surface 902A and the second inner surface 902B into the heat-dissipation channel 903. In this embodiment, a width of the fan 92 corresponds to a width of the base 90 when assembled with circuit boards, and the heat-dissipation channel 903 of the base 90 is aligned with an axis of the fan 92. The heat-dissipation fins 905 may be integrally formed with the base 90. In addition, the first outer surface 901A corresponds to the first inner surface 902A, and the second outer surface 901B corresponds to the second inner surface 902B. When the hydrogen generator E with the sound-proof shield is operating, electronic components on the first circuit board 91A and the second circuit board 92B disposed on the outer surface of the base 90 also operate and generate heat, and the heat is rapidly conducted to the heat-dissipation fins 905 disposed on the inner surface of the base 90. Furthermore, when the fan 92 operates, the fan 92 not only introduces ambient air into the heat-dissipation channel to contact the heat-dissipation fins 905 and carry away heat therefrom, but the ambient air can also directly contact the electronic components on the first circuit board 91A and the second circuit board 92B to carry away heat generated by the electronic components, and a flow direction of the ambient air is indicated by arrows in Figs. 3 and 10A. Therefore, the hydrogen generator with the sound-proof shield of the present invention can effectively perform heat dissipation through the new power module without causing an excessively high operating temperature of the hydrogen generator, thereby improving operating efficiency.

In summary, the hydrogen generator with the sound-proof shield of the present invention can block sound generated by the gas comprising hydrogen flowing in the device through the sound-proof shield, thereby improving user experience. In addition, the hydrogen generator with the sound-proof shield includes a filter channel device and a condenser that are individually detachable, thereby improving convenience and installation efficiency. Moreover, the condenser of the hydrogen generator with the sound-proof shield can effectively increase a condensation path and heat-dissipation performance through a single extendable flow path and heat-dissipation elements, thereby enhancing condensation and filtering efficiency. Furthermore, the hydrogen generator with the sound-proof shield includes a new power module that effectively dissipates heat from circuit boards through the base including the heat-dissipation channel, thereby improving operating efficiency.

With the examples and explanations mentioned above, the features and spirits of the invention are hopefully well described. More importantly, the present invention is not limited to the embodiment described herein. Those skilled in the art will readily observe that numerous modifications and alterations of the device may be made while retaining the teachings of the invention. Accordingly, the above disclosure should be construed as limited only by the metes and bounds of the appended claims.

## Claims

1. A hydrogen generator comprising:
a water tank configured to contain water;
an electrolysis module configured to receive the water from the water tank and electrolyze the water to generate and output a gas comprising hydrogen;
a humidifying module stacked above the water tank and configured to humidify the gas comprising hydrogen, the humidifying module having a humidifying chamber configured to contain supplement water;
a diffusing device disposed in the humidifying chamber and configured to diffuse the gas comprising hydrogen output by the electrolysis module; and
a sound-proof shield disposed in the humidifying chamber and having a sound-proof cavity configured to accommodate the diffusing device, the sound-proof shield comprising a connecting tube isolated from the sound-proof cavity, a top portion of the sound-proof shield comprising a gas outlet communicating the sound-proof cavity with the humidifying chamber, the connecting tube being in fluid communication with the diffusing device and configured to guide the gas comprising hydrogen generated by the electrolysis module into the diffusing device; wherein the gas comprising hydrogen flows through the diffusing device to the sound-proof cavity, and the gas comprising hydrogen in the sound-proof cavity flows through the gas outlet to the humidifying chamber.

2. The hydrogen generator of claim 1, further comprising a filter sheet disposed outside the sound-proof shield and configured to cover the gas outlet.

3. The hydrogen generator of claim 2, further comprising a securing structure configured to contact and abut against the filter sheet, thereby attaching the filter sheet to the sound-proof shield and covering the gas outlet.

4. The hydrogen generator of claim 2, wherein the water level of the supplement water in the humidifying chamber is lower than a mounting position of the filter sheet.

5. The hydrogen generator of claim 1, wherein a gap is formed between an outer wall of the sound-proof shield and an inner wall of the humidifying module.

6. The hydrogen generator of claim 1, wherein the diffusing device further comprises a plurality of micro-holes to allow the gas comprising hydrogen to pass through the micro-holes into the sound-proof cavity and to form a plurality of microbubbles in the supplement water.

7. The hydrogen generator of claim 1, further comprising a filter channel device, a condenser, and an integrated flow channel device, the filter channel device being configured to receive and filter the gas comprising hydrogen, the condenser being stacked above the water tank and connected to the filter channel device, the condenser being configured to receive and condense the gas comprising hydrogen output by the filter channel device, the integrated flow channel device being stacked above the water tank and communicating the condenser and the humidifying module, the integrated channel device being configured to receive the condensed gas comprising hydrogen and output thecondensed gas comprising hydrogen to the humidifying module.

8. The hydrogen generator of claim 7, wherein the integrated flow channel device comprises a supplement water channel, and the sound-proof shield comprises a supplement water passage communicating the supplement water channel and the sound-proof cavity, the supplement water is replenished into the sound-proof cavity through the supplement water channel and the supplement water passage.

9. The hydrogen generator of claim 1, further comprising a power module disposed above the water tank, the power module being electrically connected to the electrolysis module and configured to supply electric energy to the electrolysis module to electrolyze the electrolysis water.

10. The hydrogen generator of claim 9, wherein the power module comprises a base in a cuboid shape and a plurality of heat-dissipation fins, the base has a heat-dissipation channel and an inner surface, and the heat-dissipation fins extend inward from the inner surface into the heat-dissipation channel.

11. The hydrogen generator of claim 1, further comprising an atomizer in gas communication with the humidifying module, the atomizer receiving the gas comprising hydrogen from the humidifying module and selectively generating an atomized gas to be mixed with the gas comprising hydrogen to form a health-care gas.

12. A hydrogen generator comprising:
a water tank having a space configured to contain electrolysis water;
an electrolysis module configured to receive the electrolysis water from the water tank and electrolyze the electrolysis water to generate and output a gas comprising hydrogen;
a humidifying module stacked above the water tank and configured to humidify the gas comprising hydrogen, the humidifying module having a humidifying chamber configured to contain supplement water;
a sound-proof shield disposed in the humidifying chamber and having a sound-proof cavity, the gas comprising hydrogen output by the electrolysis module being guided into the sound-proof cavity; and
a power module disposed above the water tank and electrically connected to the electrolysis module, the power module being configured to supply electric energy to the electrolysis module and comprising a base, a first circuit board, a second circuit board, and a fan, the base having a first outer surface and a second outer surface opposite to each other and comprising a heat-dissipation channel located between the first outer surface and the second outer surface, the first circuit board and the second circuit board being respectively disposed on the first outer surface and the second outer surface, and the fan being disposed at one end of the base and configured to introduce ambient air into the heat-dissipation channel.

13. The hydrogen generator of claim 12, wherein the base has a first inner surface and a second inner surface opposite to each other and comprises a plurality of heat-dissipation fins, the heat-dissipation fins respectively extend inward from the first inner surface and the second inner surface into the heat-dissipation channel.

14. The hydrogen generator of claim 12, wherein the electrolysis module is disposed in the space of the water tank, the water tank comprises a tank body and a cover, the electrolysis module has an electrolysis module body, the cover comprises a first fixing portion, the electrolysis module body comprises a second fixing portion toward the cover and connected to the first fixing portion, thereby suspending the electrolysis module from the cover.

15. The hydrogen generator of claim 14, wherein the cover comprises a first positioning structure, the electrolysis module body comprises a second positioning structure corresponding to the first positioning structure, and when the electrolysis module and the cover are connected to each other through the first fixing portion and the second fixing portion to suspend the electrolysis module from the cover, the first positioning structure is coupled to the second positioning structure.

16. The hydrogen generator of claim 14, wherein the tank body forms a plurality of third positioning structures at a bottom of the space, a bottom of the electrolysis module body comprises a plurality of fourth positioning structures corresponding to the third positioning structures, and when the electrolysis module is disposed in the space, the third positioning structures are movably coupled to the fourth positioning structures.
